# EUROPEAN PATENT APPLICATION

(11) **EP 4 265 202 A1**
(43) Date of publication of application: **25.10.2023**
(21) Application number: 21905234.7
(22) Date of filing: 09.10.2021
(51) Int. Cl.: A61B 17/22

(54) **THROMBUS REMOVING DEVICE**

(30) Priority: 18.12.2020 CN 202011513736
(71) Applicant: Shanghai Bluevascular Medtech Co., Ltd., Shanghai 201321 (CN)
(72) Inventor: FAN, Yaming, Shanghai 201321 (CN); ZHANG, Guanyi, Shanghai 201321 (CN); WANG, Jiahao, Shanghai 201321 (CN); YUAN, Zhenyu, Shanghai 201321 (CN); MIAO, Zhenghua, Shanghai 201321 (CN)
(74) Representative: Lavoix
(86) International application number: PCT/CN2021/122726
(87) International publication number: WO 2022/127305

(57) **Abstract**

The invention provides a thrombectomy apparatus (1000) including a balloon catheter (1200), a transmission tube (1400) and a sheath (1500). The balloon catheter (1200) is slidably inserted in the transmission tube (1400), and the sheath (1500) is sleeved over the transmission tube (1400). The distal and proximal ends of the balloon catheter (1200) are respectively connected to a balloon (1100) and a perfusion unit (1800). The transmission tube (1400) is rotatably connected to the thrombus aspiration head (1300). The distal end of the transmission tube (1400) is fixedly connected to a thrombus fragmentation cutter (1410). The thrombus fragmentation cutter (1410) is accommodated in the thrombus aspiration head (1300). The proximal end of the transmission tube (1400) is connected to the driving unit (1420). The proximal end of the sheath (1500) is connected to the aspiration unit (1700). The space between the outer wall of the transmission tube (1400) and the inner wall of the sheath (1500) serves as a thrombus aspiration channel (1750) that is in communication with the thrombus aspiration head (1300). A filling channel (1230) is formed in the balloon catheter (1200). The perfusion unit (1800) is configured to inject the first liquid into or draw the first liquid out of the balloon (1100) through the filling channel (1230) to expand or contract the balloon (1100). The perfusion unit (1800) is further configured to provide the second liquid to an area between the balloon (1100) and the thrombus aspiration head (1300). The aspiration unit (1700) is configured to aspirate the thrombus into the thrombus aspiration head (1300) through the thrombus aspiration channel (1750). The driving unit (1420) is configured to drive the thrombus fragmentation cutter (1410) to cut the thrombus into thrombus fragments that is to be aspirated out by the aspiration unit (1700) through the thrombus aspiration channel (1750).

## Description

### TECHNICAL FIELD

The present invention relates to the technical field of medical instruments and, more specifically, to a thrombectomy apparatus.

### BACKGROUND

With the development of technologies, a percutaneous mechanical thrombectomy (PMT) apparatus has been developed in recent years. It is a set of instruments for treating acute and subacute thrombosis in blood vessels. The apparatus removes thrombi in blood vessels by means of dissolution, fragmentation, and aspiration, to restore blood circulation and valvular function. PMT is an interventional intracavitary thrombectomy apparatus capable of quickly removing blood clots and restoring blood flow and valvular function.

US patent publication US2014/0088610 A1 discloses a structure of a mechanical thrombectomy apparatus, which has a catheter with multiple side holes on its tip. An independent conduit for liquid delivery and perfusion is provided in the catheter, and the conduit is formed with multiple orifices on its tip. When the apparatus starts working, high-pressure saline is perfused into the independent conduit and ejected from the orifices on the tip of the conduit. Bernoulli Effect is produced by interaction between the high-speed saline jets and the side holes on the tip of the catheter, and thus thrombi are aspirated into the catheter via the side holes. Meanwhile, large pieces of the aspirated thrombi are fragmented by the high-speed saline jets, and are expelled through aspiration by a negative pressure. After the thrombectomy procedure, thrombolytic drugs can be perfused through the catheter into the blood vessels to reduce residual thrombi. This patent solves the problem of sucking and expelling thrombus from the body. However, the thrombus is cut by water flow, the aspiration effect is not good for old thrombus, and it is difficult to cut thrombus with high pressure normal saline into a suitable size for aspiration by the aspiration orifice, which results in a low aspiration efficiency. Moreover, the thrombus fragmenting process is carried out outside the catheter. The high-speed injection of normal saline destroys the red blood cells in the blood, and these destroyed red blood cells are not all aspirated from the body, which is easy to cause complications such as hematuria and renal failure.

WO2019001245A1 discloses a mechanical thrombus removal device, which mainly includes a thrombus fragmentation system, a thrombus aspiration system, and a drug perfusion system. When the mechanical thrombus removal device is operated, the drug perfusion system is used to inject thrombolytic drugs to the lesion site, and then the thrombus fragmentation system and the thrombus aspiration system are activated after the drug fully reacts with the lesion. The thrombus near the thrombus aspiration head is aspirated into the catheter under the action of the negative pressure generated by the peristaltic pump in the thrombus aspiration system, is cut by the thrombus fragmentation cutter of the thrombus fragmenting system, and is drawn out by the thrombus aspiration system from the body. The drug perfusion system of this device requires a syringe configured to perfuse drugs to the lesion intermittently, thereby limiting the dissolving effect. Moreover, in this process, the drug perfused into the blood vessel may easily cause the thrombus to flow to the lungs with the blood. The thrombus fragmenting process is carried out inside the catheter to avoid hematuria. However, a long-time aspiration may cause patients to lose a lot of blood, which brings danger to some patients with poor physical conditions.

In view of the deficiencies of the existing mechanical thrombectomy apparatuss, those skilled in the art have been looking for solutions to the problem on how to improve the efficiency of thrombectomy and prevent thrombus from drifting.

### SUMMARY OF THE INVENTION

An objective of the present invention is to provide a thrombectomy apparatus, which can improve the efficiency of thrombectomy and prevent thrombus from drifting, so as to improve efficacy of thrombectomy.

To achieve the above objective, the present invention provides a thrombectomy apparatus, comprising a balloon catheter, a transmission tube, and a sheath; wherein the balloon catheter is slidably inserted in the transmission tube, and the sheath is sleeved over the transmission tube;
wherein the balloon catheter has a distal end connected to a balloon, and a proximal end connected to a perfusion unit; the sheath has a distal end connected a thrombus aspiration head; the transmission tube is rotatably connected to the thrombus aspiration head; the transmission tube has a distal end fixedly connected with a thrombus fragmentation cutter accommodated in the thrombus aspiration head, and a proximal end connected to a driving unit; and the sheath further has a proximal end connected to an aspiration unit;
wherein a filling channel is formed in the balloon catheter; the perfusion unit is configured to inject a first liquid into or draw the first liquid out of the balloon through the filling channel to expand or contract the balloon; a first perfusion channel is formed in the balloon catheter; the first perfusion channel is provided with at least one first perfusion opening located between the balloon and the thrombus aspiration head; and the perfusion unit is further configured to provide a second liquid to an area between the balloon and the thrombus aspiration head through the first perfusion channel and the first perfusion opening;
wherein a space that serves as a thrombus aspiration channel is reserved between an outer wall of the transmission tube and an inner wall of the sheath; the thrombus aspiration channel is in communication with the thrombus aspiration head; the aspiration unit is configured to aspirate the thrombus into the thrombus aspiration head; the driving unit is configured to drive the thrombus fragmentation cutter to cut the thrombus into thrombus fragments; and the aspiration unit is configured to aspirate the thrombus fragments out through the thrombus aspiration channel.

Optionally, the balloon catheter is a multi-channel tube, and a guide wire channel is formed at a center of the balloon catheter.

Optionally, the filling channel and the first perfusion channel are symmetrical with each other about a central axis of the guide wire channel.

Optionally, an outer tube is sleeved over the sheath; at least one second perfusion channel is formed in the outer tube along an axial direction thereof; the second perfusion channel has at least one second perfusion opening located at a distal end of the outer tube; and the perfusion unit is further configured to provide a third liquid to an area between the balloon and the thrombus aspiration head through the second perfusion channel and the second perfusion opening.

Optionally, the distal end of the outer tube is horn-shaped.

Optionally, the perfusion unit comprises a first perfusion subunit and a second perfusion subunit; the first perfusion subunit is configured to inject the first liquid into the balloon or drawn out the first liquid from the balloon, and the second perfusion subunit is configured to provide the second liquid to the area.

Optionally, the first perfusion subunit comprises a first adaptor, a first infusion tube and a syringe that is provided therein with the first liquid; the first infusion tube has a distal end that is in communication with the filling channel via the first adaptor, and a proximal end that is in communication with the syringe.

Optionally, the second perfusion subunit comprises a second adaptor, a second infusion tube, and a perfusion liquid bag that is provided therein with the second liquid; the second infusion tube has a distal end that is in communication with the first perfusion channel via the second adaptor, and a proximal end that is in communication with the perfusion liquid bag; and a first peristaltic pump is provided to the second infusion tube.

Optionally, a plurality of thrombus aspiration holes are formed in a distal end and/or a sidewall of the thrombus aspiration head.

Optionally, the aspiration unit comprises a third adaptor, a third infusion tube, and a thrombus collection bag; the third infusion tube has a distal end that is in communication with the thrombus aspiration channel via the third adaptor, and a proximal end that is communication with the thrombus collection bag; a second peristaltic pump is provided to the third infusion tube.

Optionally, the first liquid is a developing solution, and the second liquid is a thrombolytic drug solution and/or normal saline.

The present invention further provides a thrombectomy apparatus, comprising a balloon catheter, a transmission tube, a sheath, and an outer tube; wherein the balloon catheter is slidably inserted in the transmission tube, the sheath is sleeved over the transmission tube, and the outer tube is sleeved over the sheath;
wherein the balloon catheter has a distal end connected to a balloon, and a proximal end connected to a perfusion unit; the sheath has a distal end connected a thrombus aspiration head; the transmission tube is rotatably connected to the thrombus aspiration head; the transmission tube has a distal end fixedly connected with a thrombus fragmentation cutter accommodated in the thrombus aspiration head, and a proximal end connected to a driving unit; and the sheath further has a proximal end connected to an aspiration unit;
wherein a filling channel is formed in the balloon catheter; the perfusion unit is configured to inject a first liquid into or draw the first liquid out of the balloon through the filling channel to expand or contract the balloon; a plurality of second perfusion channels are formed in an wall of the outer tube along an axial direction thereof; a second perfusion opening is formed at a distal end of the outer tube; and the perfusion unit is further configured to provide a second liquid to an area between the balloon and the thrombus aspiration head through the second perfusion channel and the second perfusion opening;
wherein a space that serves as a thrombus aspiration channel is reserved between an outer wall of the transmission tube and an inner wall of the sheath; the thrombus aspiration channel is in communication with the thrombus aspiration head; the aspiration unit is configured to aspirate the thrombus into the thrombus aspiration head; the driving unit is configured to drive the thrombus fragmentation cutter to cut the thrombus into thrombus fragments; and the aspiration unit is configured to aspirate the thrombus fragments out through the thrombus aspiration channel.

In the thrombectomy apparatus provided by the present invention, the perfusion unit is configured to inject a first liquid into or draw the first liquid out of the balloon through the filling channel to expand or contract the balloon, so that the balloon and the inner wall of the blood vessel distal to the thrombus can be closely attached with each other. Therefore, the balloon can block the blood vessel distal to the thrombus during the thrombectomy, preventing thrombus fragments from escaping from the blood vessel distal to the thrombus. The perfusion unit is further configured to provide a second liquid to the area between the thrombus aspiration head and the balloon. The second liquid may be a thrombolytic drug solution and/or normal saline, etc., which can be used to flush the thrombus so as to apply an additional impact on it, thereby facilitate the detachment of the thrombus from the inner wall of the blood vessel. The thrombolytic drug solution can promote the dissolution of the thrombus, so that the operation time can be reduced, and the efficiency of the thrombectomy is improved. During the process of aspiration of the thrombus fragments, the thrombolytic drug solution and/or normal saline perfused in the vicinity of the thrombus aspiration head by the perfusion unit surrounds the thrombus fragments so as to be aspirated together by the aspiration unit, which can prevent the blood in the blood vessel from being aspirated by the aspiration unit excessively so as to reduce the blood loss of the patient.

### BRIEF DESCRIPTION OF THE DRAWINGS

FIG. 1 is a schematic diagram showing of a thrombectomy apparatus according to Embodiment 1 of the present invention;
FIG. 2 is a sectional view of the thrombectomy apparatus along the A-A section according to Embodiment 1 of the present invention;
FIG. 3 is a sectional view of a thrombus aspiration head according to Embodiment 1 of the present invention;
FIG. 4 is a schematic diagram showing a balloon catheter according to Embodiment 1 of the present invention;
FIG. 5 is a sectional view of the balloon catheter according to Embodiment 1 of the present invention;
FIG. 6 is a front view of the balloon catheter according to Embodiment 1 of the present invention;
FIG. 7 is a top view of the balloon catheter according to Embodiment 1 of the present invention;
FIG. 8 is a schematic diagram showing of a thrombectomy apparatus according to Embodiment 2 of the present invention;
FIG. 9 is a sectional view of the thrombectomy apparatus along the A-A section according to Embodiment 2 of the present invention;
FIG. 10 is a schematic diagram showing an outer sheath according to Embodiment 2 of the present invention;
FIG. 11 is a sectional view of the thrombectomy apparatus along the A-A section according to Embodiment 3 of the present invention; and
FIG. 12 is a sectional view of the balloon catheter according to Embodiment 3 of the present invention.

### List of reference signs:

1000, thrombectomy apparatus; 1100, balloon; 1110, visualization ring; 1200, balloon catheter; 1210, filling opening; 1220, first perfusion opening; 1230, filling channel; 1240, first perfusion channel; 1250, guide wire channel; 1300, thrombus aspiration head; 1310, thrombus aspiration hole; 1400, transmission tube; 1410, thrombus fragmentation cutter; 1420, driving unit; 1500, sheath; 1600, outer tube; 1610, second perfusion channel; 1620, second perfusion opening; 1700, aspiration unit; 1710, third adaptor; 1720, third infusion tube; 1730, second peristaltic pump; 1740, thrombus collection bag; 1750, thrombus aspiration channel; 1800, perfusion unit; 1810, first perfusion subunit; 1811, first adaptor; 1812, first infusion tube; 1813, syringe; 1820, second perfusion subunit; 1821, second adaptor; 1822, second infusion tube; 1823, first peristaltic pump ; 1824, perfusion liquid bag;
2000, guide wire; 2100, guide wire locking unit.

### DETAILED DESCRIPTION

The specific embodiments of the present invention will be described in more detail below with reference to the schematic diagrams. Based on the following description, the advantages and features of the present invention will become clearer. It is noted that the figures are provided in a very simplified form not necessarily presented to scale, with their only intention to facilitate convenience and clarity in explaining the disclosed embodiments.

As used herein, a "proximal end" and a "distal end" are defined as follows: the "distal end" generally refers to the end of a medical device that first enters the patient during normal operation, while the "proximal end" generally refers to the end of the medical device close to the operator during the normal operation.

A thrombus is a small piece of clot formed on the surface of the tissue where experiences exfoliation or repair on the inner wall of the blood vessel. Thrombus consists of insoluble fibrin, deposited platelets, accumulated white blood cells, and trapped red blood cells. Thrombus may slow down the blood flow and cause whirlpools in the blood flow, which may cause discomfort to the human body, endanger the survival of the limbs and even threaten the safety of life.

The thrombectomy apparatus is an apparatus configured to remove blood clots in blood vessels by means of dissolving, fragmentation, and aspiration, so as to improve blood circulation.

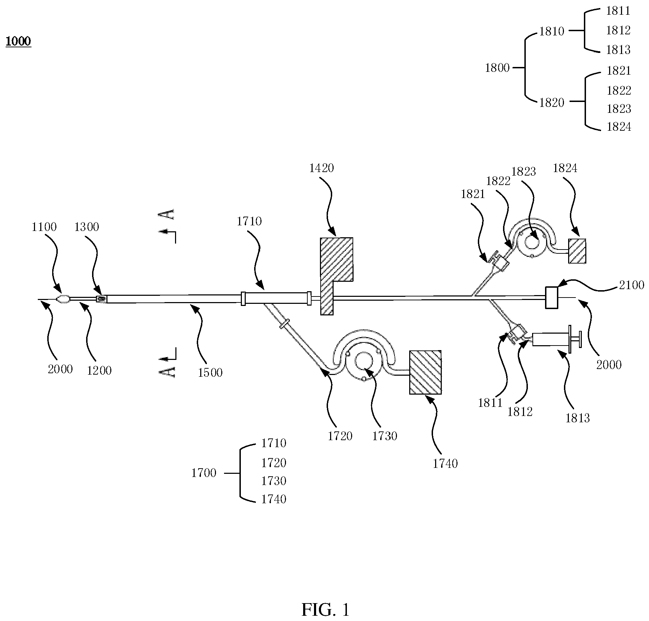

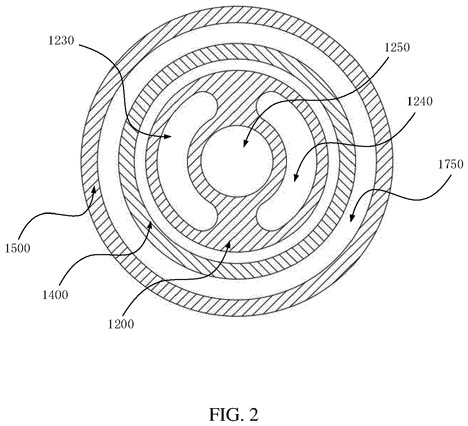

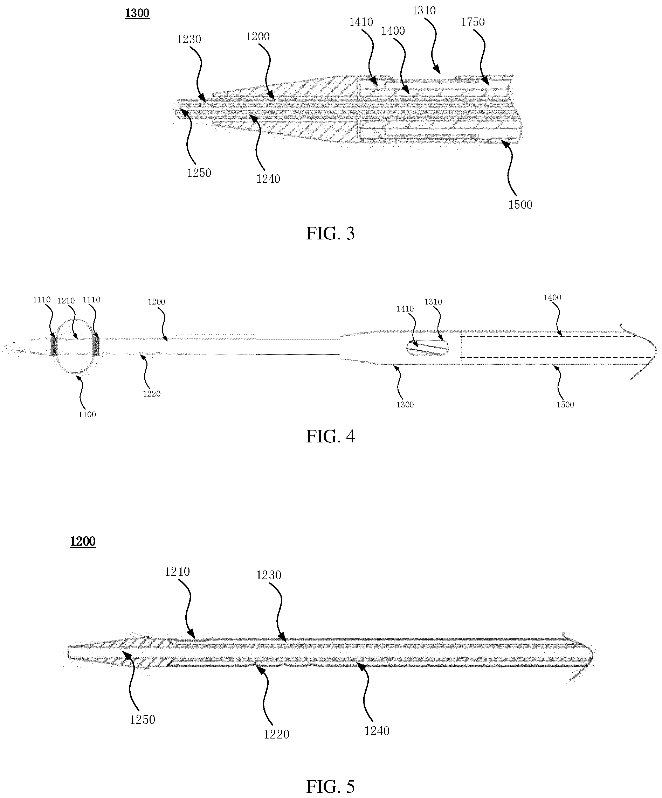

FIG. 1 is a schematic diagram showing of a thrombectomy apparatus according to the present embodiment, Fig. 2 is a sectional view of the thrombectomy apparatus along the A-A section according to the present embodiment, and FIG. 3 is a sectional view of a thrombus aspiration head according to the present embodiment. As shown in FIGs. 1, 2 and 3, the thrombectomy apparatus 1000 includes a balloon catheter 1200, a transmission tube 1400, and a sheath 1500. The balloon catheter 1200 is slidably inserted in the transmission tube 1400, and the sheath 1500 is sleeved over the transmission tube 1400.

The distal and proximal ends of the balloon catheter 1200 are respectively connected with a balloon 1100 and a perfusion unit 1800. The distal end of the sheath 1500 is connected with a thrombus aspiration head 1300, and the transmission tube 1400 is rotatably connected with the thrombus aspiration head 1300. A thrombus fragmentation cutter 1410 is fixed at the distal end of the transmission tube 1400 and is accommodated in the thrombus aspiration head 1300. The distal end of the transmission tube 1400 is connected to the thrombus aspiration head 1300, and the distal end of the sheath 1500 is also connected to the thrombus aspiration head 1300. In this way, there is no relative slip in axial direction between the transmission tube 1400 and the sheath 1500. The proximal end of the transmission tube 1400 is connected to the driving unit 1420, and the proximal end of the sheath 1500 is connected to the aspiration unit 1700. The space between the outer wall of the transmission tube 1400 and the inner wall of the sheath 1500 serves as a thrombus aspiration channel 1750 that is in communication with the thrombus aspiration head 1300. The aspiration unit 1700 is configured to aspirate the thrombus into the thrombus aspiration head 1300, the driving unit 1420 is configured to drive the thrombus fragmentation cutter 1410 to cut the thrombus into thrombus fragments, and thus the thrombus fragments can be aspirated by the aspiration unit 1700 from the body through the thrombus aspiration channel 1750.

Referring to FIGs. 1, 2 and 3, a plurality of thrombus aspiration holes 1310 are formed in the sidewall of the thrombus aspiration head 1300, and configured in such a way that thrombus can be aspirated into the thrombus aspiration head 1300 through the thrombus aspiration holes 1310. The thrombus fragmentation cutter 1410 has a tubular pierced structure with at least one blade having a cutting edge facing the thrombus aspiration hole 1310. Therefore, the thrombus can be cut under the action of both the thrombus fragmentation cutter 1410 and the thrombus aspiration hole 1310. Since the thrombus aspiration channel 1750 is in communication with the thrombus aspiration head 1300, the aspiration unit 1700 provides negative pressure inside the thrombus aspiration head 1300 when aspirating thrombus fragments so that the thrombus can be aspirated into the thrombus aspiration head 1300 via the thrombus aspiration hole 1310. As a result, the thrombus can be cut by the thrombus fragmentation cutter 1410, driven by the driving unit 1420, inside the thrombus aspiration head 1300.

Optionally, the driving unit 1420 may be a rotating motor. The thrombus aspiration head 1300 can be made of metal or a composite material including medical polymer and metal, and the transmission tube 1400 can be made of metal or a composite material including medical polymer and metal.

The balloon catheter 1200 defines a filling channel 1230 therein. The filling channel 1230 is configured in such a way that a first liquid is allowed to be filled into or drawn out from the balloon 1100 through the filling channel 1230 by the perfusion unit 1800 to expand or contract the balloon 1100. The perfusion unit 1800 also configured to provides a second liquid to the vicinity of the thrombus aspiration head 1300. Specifically, the vicinity of the thrombus aspiration head 1300 is the area between the balloon 1100 and the thrombus aspiration head 1300.

In an embodiment of the present invention, the first liquid is a developing solution. When the developing liquid enters the balloon 1100, the balloon 1100 can be easily shown on the medical imaging equipment, which makes it convenient for the operator to observe.

In another embodiment of the present invention, the first liquid and the second liquid are both normal saline. The normal saline is perfused at the lesion area during the operation so as to reduce the blood loss of the patient in the process of aspiration of the thrombus fragments by the aspiration unit 1700.

In another embodiment of the present invention, the second liquid is a thrombolytic drug solution in which a thrombolytic drug (for example, urokinase) is dissolved, so that the thrombolytic drug solution can dissolve the thrombus. The area between the balloon 1100 and the thrombus aspiration head 1300 is the lesion area (where the thrombus is located). By perfusing the thrombolytic drug solution to the lesion area, the thrombolytic drug solution can dissolve the thrombus and reduce the size of the thrombus. As a result, the operation time can be reduced, and the efficiency of thrombectomy can be improved.

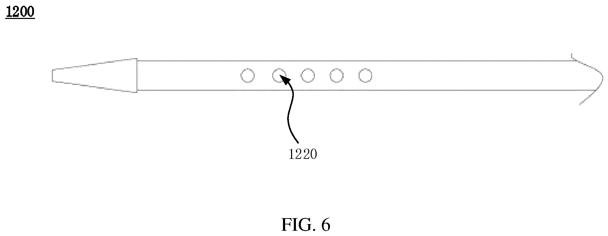

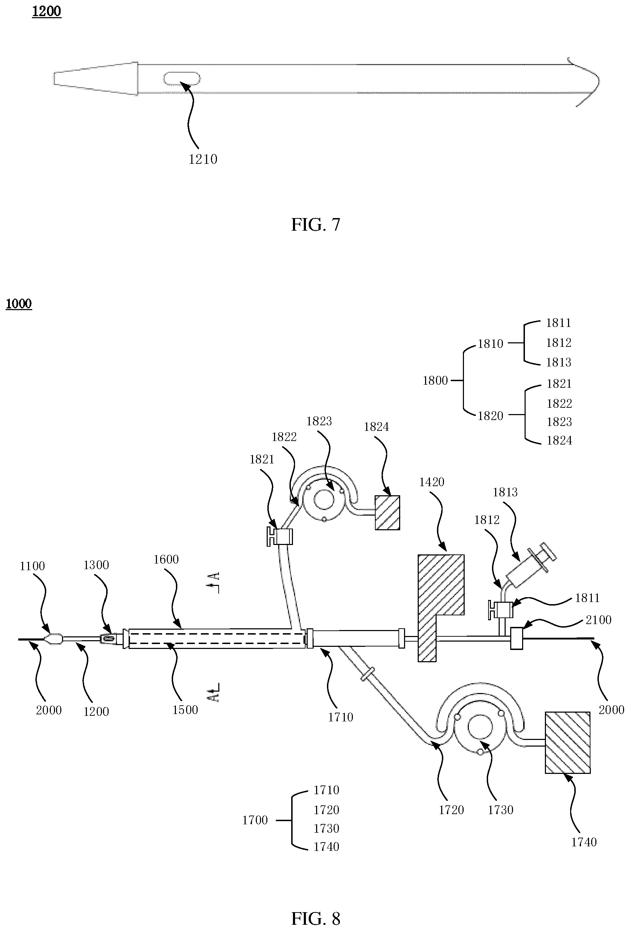

FIG. 4 is a schematic diagram showing a balloon catheter according to the present embodiment, FIG. 5 is a sectional view of the balloon catheter according to the present embodiment; FIG. 6 is a front view of the balloon catheter according to the present embodiment; FIG. 7 is a top view of the balloon catheter according to the present embodiment. As shown in FIGs. 4 to 7, in order to ensure that the perfusion unit 1800 can provide a second liquid to the area between the balloon 1100 and the thrombus aspiration head 1300. The balloon catheter 1200 is a multi-channel tube. The balloon catheter 1200 further defines a first perfusion channel 1240 therein. The first perfusion channel 1240 is formed in the wall of the balloon catheter 1200 and extends axially. At least one first perfusion opening 1220 is formed in a portion of the wall of balloon catheter 1200 between the balloon 1100 and the thrombus aspiration head 1300, and is in communication with the first perfusion channel 1240. As a result, the second liquid can be provided to the vicinity of the thrombus aspiration head 1300 through the first perfusion channel 1240 by the perfusion unit 1800. The number of the first perfusion opening 1220 includes but is not limited to one, two, three or four, which can be selected according to the requirement of the technology in the art on how much the second liquid shall be perfused. It should be known that the caliber of the first perfusion opening 1220 will also affect the perfusion volume of the second liquid. Those skilled in the art can adjust the infusion volume of the second liquid by changing the caliber of the first perfusion opening 1220 and the number of the first perfusion opening 1220.

Please continue to refer to FIG. 1, the perfusion unit 1800 optionally includes a first perfusion subunit 1810 and a second perfusion subunit 1820. The first perfusion subunit 1810 is configured to inject the first liquid into the balloon or drawn out the first liquid from the balloon, and the second perfusion subunit 1820 is configured to provide the second liquid into the body. That is, the second perfusion subunit 1820 communicates with the first perfusion opening 1220 through the first perfusion channel 1240.

The second perfusion subunit 1820 includes a second adaptor 1821, a second infusion tube 1822, and a perfusion liquid bag 1824. The distal end of the second infusion tube 1822 is in communication with the first perfusion channel 1240 via the second adaptor 1821. The proximal end of the second infusion tube 1822 is in communication with the perfusion liquid bag 1824. The second infusion tube 1822 is provided with a first peristaltic pump 1823. In this way, the second liquid (e.g., thrombolytic drug solution and/or normal saline) in the perfusion liquid bag 1824 can be delivered to the first perfusion opening 1220 by the first peristaltic pump 1823 to flow out. It should be understood that the second adaptor 1821 is provided therein with a one-way valve configured to prevent blood from flowing back into the perfusion liquid bag 1824. It should be understood that the first peristaltic pump 1823 can be replaced with a syringe 1813, and the surgeon can manipulate the syringe 1813 to manually inject the second liquid into the lesion area.

Please continue to refer to FIGs 2, 5 and 7, a filling opening 1210 is formed at the distal end of the balloon catheter 1200, which is in positional correspondence with the balloon 1100. The filling opening 1210 is in communication with the filling channel 1230 that is formed in the wall of the balloon catheter 1200 and extends axially. In other words, the first perfusion subunit 1810 is in communication with the filling opening 1210 via the filling channel 1230, and thus can inject the first liquid into the balloon 1100 or drawn out the first liquid from the balloon 1100 to expand or contract the balloon 1100.

Please continue to refer to FIG. 1, the first perfusion subunit 1810 includes a first adaptor 1811, a first infusion tube 1812, and a syringe 1813 in which the first liquid is provided. The distal end of the first infusion tube 1812 is in communication with the filling channel 1230 via the first adaptor 1811. The proximal end of the first infusion tube 1812 is in communication with the syringe 1813. The first adaptor 1811 is provided therein with a one-way valve. It should be understood that it is not necessary to include the first infusion tube 1812 in the first perfusion subunit 1810. The first infusion tube 1812 is so provided that the syringe 1813 can be moved in a larger range. As a result, the syringe 1813 can be placed in more positions, which makes it convenient for performing the operation.

It should be understood that, during the operation of thrombectomy, the surgeon injects the first liquid into the balloon 1100 to expand the balloon 1100 at a location distal to the thrombus. The balloon 1100 becomes larger during the expansion thereof so that the surface of the balloon 1100 can be closely attached to the inner wall of the blood vessel, and the balloon 1100 isolates the lesion area from the tissues distal thereto. During the operation, thrombus fragments can be prevented from entering the blood circulation from areas distal to the lesion area so as to avoid complications, such as hematuria and renal failure, caused therefrom. Moreover, it can also prevent pulmonary embolism resulting from the thrombus fragments which are delivered into the lungs by the blood flow.

The two ends of the balloon 1100 are fixed on the balloon catheter 1200 by the visualization ring 1110. In other words, the two ends of the balloon 1100 are wound on the balloon catheter 1200 by the visualization ring 1110, and the visualization ring 1110 is easy to be shown on medical imaging equipment. In this way, it is convenient for the surgeon to know the accurate position of the balloon 1100 in the blood vessel.

Preferably, the balloon 1100 is made of medical silicone, latex or elastomeric polymer. The balloon catheter 1200 is made of medical polymer or a composite material including medical polymer and metal. The balloon catheter 1200 is a flexible so that it can bend following the shape of the blood vessel.

It should be known that the balloon catheter 1200 can move inside the transmission tube 1400 in the axial direction because the balloon catheter 1200 is slidably inserted in the transmission tube 1400. In this way, the distance between the thrombus aspiration head 1300 and the balloon 1100 provided at the distal end of the balloon catheter 1200 can be adjusted by moving the balloon catheter 1200. Since the distance between the balloon 1100 and the thrombus aspiration head 1300 is adjustable, the surgeon can adjust the distance between the balloon 1100 and the thrombus aspiration head 1300 during the operation of thrombectomy according to the size of the thrombus so as to adapt to the thrombus.

In another embodiment of the present invention, the surgeon can also move the balloon catheter 1200 back and forth to drive the balloon 1100 to move back and forth in the blood vessel, so as to draw the thrombus to the vicinity of the thrombus aspiration head 1300. Therefore, the thrombus can be aspirated and cut in the thrombus aspiration head 1300.

There may be a long-term thrombus attached to the inner wall of the blood vessel. In the later stage of the operation, the surgeon can adjust the distance between the balloon 1100 and the thrombus aspiration head 1300 to drive the balloon 1100 move back and forth in the blood vessel, during which the balloon 1100 scratches the inner wall of the blood vessel, so that the balloon 1100 can peel off the attached thrombus during the process of moving back the balloon 1100. Therefore, the thrombus peeled off from the inner wall of the blood vessel can be discharged from the body by passing through the thrombus aspiration head 1300 and the thrombus aspiration channel 1750. As a result, the efficacy of thrombectomy is improved.

Optionally, a guide wire channel 1250 is formed in the center of the balloon catheter 1200. The filling channel 1230 and the first perfusion channel 1240 are both axially formed in the wall of the balloon catheter 1200, and are symmetrical with each other about the center axis of the guide wire channel 1250. It should be understood that there are many other arrangements of the filling channel 1230 and the first perfusion channel 1240 in the wall of the balloon catheter 1200, and they may not be symmetrical, and may have different cross-sectional sizes, which are not limited here.

The guide wire channel 1250 is configured in such a way that the guide wire 2000 can be inserted therethrough. In an embodiment of the present invention, a guide wire locking unit 2100 is generally provided at the proximal end of the balloon catheter 1200. The guide wire channel 1250 can be adjusted to open or close by locking or unlocking the guide wire locking unit 2100. When the guide wire locking unit 2100 is locked, the proximal end of the balloon catheter 1200 is locked, so that the guide wire 2000 cannot be moved in or out. When the guide wire locking unit 2100 is unlocked, the guide wire channel 1250 opens and allows the guide wire 2000 to move in or out.

The guide wire 2000 is configured to guide the thrombectomy apparatus 1000 to a designated position. During the operation, the blood vessel is first punctured, and then one end (that is, the distal end) of the guide wire 2000 is inserted into the diseased blood vessel and passes through the thrombus, and the other end (that is, the proximal end) of the guide wire 2000 is inserted into the guide wire channel 1250 of the thrombectomy apparatus 1000 (that is, the guide wire channel 1250 in the center of the balloon catheter 1200). The front end of the thrombectomy apparatus 1000 is then guided into the blood vessel along the guide wire 2000 at the position where the thrombus is. The balloon 1100 and part of the balloon catheter 1200 provided at the front end of the thrombectomy apparatus 1000 will pass through the thrombus along the guide wire 2000, so that the balloon 1100 and the thrombus aspiration head 1300 are respectively located at the positons distal and proximal to the thrombus.

Please continue to refer to FIG. 1, the aspiration unit 1700 includes a third adaptor 1710, a third infusion tube 1720, and a thrombus collection bag 1740. The third infusion tube 1720 is in communication with the thrombus aspiration channel 1750 via the third adaptor 1710. The proximal end of the third infusion tube 1720 is in communication with the thrombus collection bag 1740. The third infusion tube 1720 is provided with a second peristaltic pump 1730.

The third adaptor 1710 is also called an intermediate adaptor. The third adaptor 1710 has a letter "Y" shape. The third adaptor 1710 has a double-pass design so that the transmission tube 1400 can pass through the third adaptor 1710 without any constraint. The distal end of the third adaptor 1710 is connected to the sheath 1500, and the Y-shaped end of the third adaptor 1710 is connected to the distal end of the third infusion tube 1720. The proximal end of the third infusion tube 1720 is in connection and communication with the thrombus collection bag 1740. The second peristaltic pump 1730 can aspirate blood mixed with thrombus fragments into the thrombus collection bag 1740 through the thrombus aspiration channel 1750. Preferably, the third adaptor 1710 is made of a medical polymer.

To better understand the thrombectomy apparatus of the present invention, the application of the thrombectomy apparatus 1000 will be described in detail below with reference to FIGs. 1 to 7. The specific process is as follows.

Before the operation starts, the first peristaltic pump 1823 is connected to the second adaptor 1821, and the first peristaltic pump 1823 of the second perfusion subunit 1820 is activated to operate until the thrombolytic drug solution in the perfusion liquid bag 1824 goes out of the first perfusion opening 1220. The thrombus aspiration head 1300 of the thrombectomy apparatus 1000 is then placed in the mixed solution containing normal saline and heparin, and the second peristaltic pump 1730 of the aspiration unit 1700 is activated to operate until the mixed solution containing normal saline and heparin is visible in the thrombus collection bag 1740. Subsequently, the thrombus aspiration head is taken out of the mixed solution containing normal saline and heparin (which has an anticoagulant effect preventing blood from clotting in the thrombus aspiration head 1300, the thrombus aspiration channel 1750, the third adaptor 1710, and the third infusion tube 1720, to avoid a failure in aspiration). The syringe 1813 in the first perfusion subunit 1810 is connected to the first adaptor 1811 by the first infusion tube 1812, so that the syringe 1813 is in communication with the filling channel 1230 and the filling opening 1210, and the aspiration starts and lasts until the balloon 1100 is no longer smaller. The one-way valve is then locked. The aim of the above operation is to empty the air in the thrombectomy apparatus 1000.

During the operation, the blood vessel is punctured, the distal end of the thrombectomy apparatus 1000 is introduced into the blood vessel along the guide wire 2000, and the thrombectomy apparatus 1000 is transferred to a positon in front of the thrombus with the aid of the medical imaging equipment scanning the visualization rings 1110 at both ends of the balloon 1100. After the first peristaltic pump 1823 injects a certain amount of thrombolytic drug into the blood vessel, the first peristaltic pump 1823 is deactivated, and then the guide wire 2000 is inserted into the guide wire channel 1250 of the balloon catheter 1200 so that the distal end of the guide wire 2000 passes through the thrombus. Subsequently, the proximal end of the balloon catheter 1200 is pushed so that the balloon catheter 1200 is moved distally along the guide wire 2000 until the balloon 1100 passes through the thrombus. In this way, the balloon 1100 and the thrombus aspiration head 1300 are respectively positioned distally and proximally to the thrombus.

Then, the syringe 1813 is operated to inject the developing solution into the balloon 1100 through the first infusion tube 1812, and the medical imaging equipment is used to check whether the outer wall of the balloon 1100 is closely attached with the inner wall of the blood vessel. If the outer wall of the balloon 1100 is closely attached with the inner wall of the blood vessel, the driving unit 1420 is activated to drive the transmission tube 1400 to drive the thrombus fragmentation cutter 1410 to rotate. Subsequently, the second peristaltic pump 1730 is activated, and then the first peristaltic pump 1823 is activated. Specifically, the first peristaltic pump 1823 delivers the normal saline in the perfusion liquid bag 1824 to a lesion area between the balloon 1100 and the thrombus aspiration head 1300 through the second infusion tube 1822, the second adaptor 1821, the first perfusion channel 1240, and the first perfusion opening 1220. The second peristaltic pump 1730 rotates so that the aspiration unit 1700 provides negative pressure to the thrombus aspiration head 1300 and the thrombus aspiration channel 1750, and part of the thrombus is therefore aspirated into the thrombus aspiration head 1300, and then the thrombus fragmentation cutter 1410 is rotated to cooperate with the thrombus aspiration hole 1310 of the thrombus aspiration head 1300, so as to cut, into fragments, the thrombus aspirated into the thrombus aspiration head 1300. The cut thrombus fragments are then aspirated into the thrombus collection bag 1740 through the thrombus aspiration channel 1750, the third adaptor and the third infusion tube 1720. At the same time, the normal saline and part of the thrombolytic drug solution injected through the first perfusion opening 1220 are also aspirated into the thrombus collection bag 1740.

During the operation, the thrombectomy apparatus 1000 can be moved back and forth along the guide wire 2000 according to the specific position of the thrombus in the blood vessel to improve the efficiency of thrombectomy. It is also possible to move back the balloon catheter 1200 so as to drive the balloon 1100 to move in the blood vessel so that the thrombus can be moved to the area in front of the thrombus aspiration head 1300 for aspiration and fragmentation, thereby improving the efficiency of thrombectomy. It is also possible to move back the balloon catheter 1200 to drive the balloon 1100 to move toward the thrombus aspiration head 1300, so as to rub the thrombus to cause the thrombus to fall.

During the operation, the thrombolytic drug solution in the perfusion liquid bag 1824 can also be replaced with normal saline or a mixture of thrombolytic drug solution and normal saline, which can prevent the aspiration unit 1700 from aspirating too much blood and causing excessive blood loss. It should be understood that the rotation speed of the first peristaltic pump 1823 and the second peristaltic pump 1730 can be matched to adjust the perfusion volume of the liquid from the second perfusion subunit 1820 and the volume of the liquid aspirated into the aspiration unit 1700.

After thrombus aspiration is completed, the driving unit 1420 is first deactivated, then the first peristaltic pump 1823 and the second peristaltic pump 1730 are successively activated, and then the developing solution in the balloon 1100 is aspirated by the syringe 1813, so that the balloon 1100 contracts accordingly. Finally, the thrombectomy apparatus 1000 and the guide wire 2000 are withdrawn from the human body together.

### Embodiment 2

In the thrombectomy apparatus provided in this embodiment, the same parts as those in Embodiment 1 will not be described here, and only the differences will be described below.

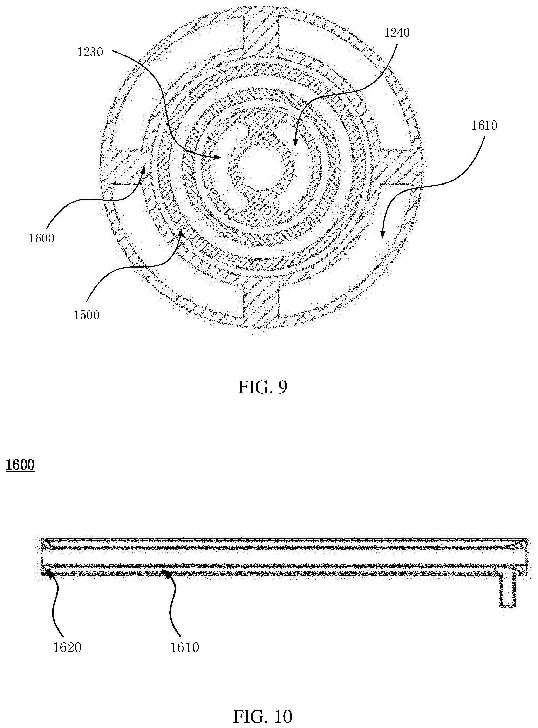

FIG. 8 is a schematic diagram showing of a thrombectomy apparatus according to the present embodiment; FIG. 9 is a sectional view of the thrombectomy apparatus along the A-A section according to the present embodiment; FIG. 10 is a schematic diagram showing an outer sheath according to the present embodiment. As shown in FIGs. 8 to 10, the difference between the present embodiment and Embodiment 1 is that the thrombectomy apparatus 1000 further includes an outer tube 1600 sleeved over the sheath 1500, and a space is reserved between the outer tube 1600 and the sheath 1500, so that the outer tube 1600 can move freely back and forth along the sheath 1500. A plurality of second perfusion channels 1610 are formed in the wall of the outer tube 1600 along the axial direction thereof. The second perfusion subunit 1820 is configured to provide the third liquid to the vicinity of the thrombus aspiration head 1300 through the second perfusion channels 1610. A second perfusion opening 1620 is formed at the distal end of the outer tube 1600. The third liquid is perfused into the area between the thrombus aspiration head 1300 and the balloon 1100 from the second perfusion opening 1620 through the second perfusion channels 1610. In detail, the vicinity of the thrombus aspiration head 1300 is the area between the balloon 1100 and the thrombus aspiration head 1300. In one embodiment, the third liquid is the same as the second liquid. The third liquid and the second liquid are thrombolytic drug liquid and/or normal saline. In another embodiment, the third liquid is different from the second liquid, the third liquid is normal saline, and the second liquid is a thrombolytic drug solution.

In an implementation of this embodiment, the thrombectomy apparatus includes two second perfusion subunits 1820, which are respectively in connection and communication with the first perfusion channel 1240 and the second perfusion channel 1610. The first perfusion channel 1240 is only perfused with thrombolytic drug solution, and the second perfusion channel 1610 is only perfused with normal saline. In this way, the infusion of thrombolytic drug solution and normal saline can be adjusted more accurately. In another implementation of this embodiment, both the first perfusion channel 1240 and the second perfusion channel 1610 can be perfused with thrombolytic drug solution and normal saline. In this way, when a large amount of normal saline needs to be perfused, sufficient perfusion can be achieved.

It should be understood that the cross-sectional area of the second perfusion channels 1610 in the outer tube 1600 is generally larger than that of the first perfusion channel 1240, and the number of the second perfusion channels 1610 is also greater than that of the first perfusion channels 1240. Based on this, the second perfusion channel 1610 can be used to provide a greater perfusion volume.

In the application of the thrombectomy apparatus 1000 of this embodiment, before the operation, the thrombolytic drug solution can be perfused only through the first perfusion channel 1240. As a result, the infusion amount of the thrombolytic drug solution is controllable so as to avoid excessive thrombolytic drug solution to be injected.

During the operation, normal saline is perfused through the second perfusion channel 1610. The large number of second perfusion channels 1610 with larger cross-sectional areas can be used for supplementing sufficient normal saline to further reduce the blood loss of the human body.

Further, the distal end of the outer tube 1600 is horn-shaped. The outer tube 1600 with a flared opening can reduce the space between the outer tube 1600 and the inner wall of the blood vessel. After the second perfusion subunit 1820 is activated, the normal saline is sprayed from the flared opening at the distal end of the outer tube 1600. As a result, it is possible to prevent the free thrombus which is not cut into small fragments in the thrombus aspiration head 1300 from flowing into the blood circulation through the space between the outer tube 1600 and the inner wall of the blood vessel.

The second perfusion channels 1610 of the outer tube 1600 may have different cross-sectional shapes. When necessary, the second perfusion channels 1610 with different cross-sectional sizes can be used to finely adjust the perfusion amount of the second liquid. It should be understood that the cross-sectional shape of the second perfusion channels 1610 includes, but is not limited to, a fan shape, a circle, or a rectangle.

Preferably, the outer tube 1600 is made of a medical polymer or a composite material including medical polymer and metal.

### Embodiment 3

In the thrombectomy apparatus provided in this embodiment, the same parts as those in Embodiments 1 and 2 will not be described here, and only the differences will be described below.

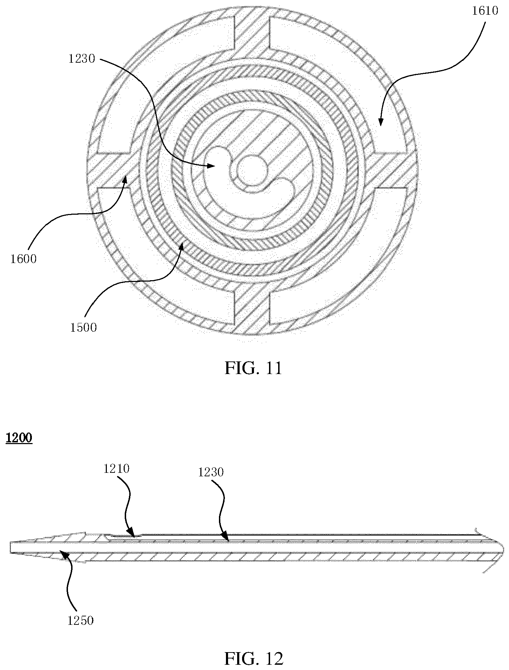

FIG. 11 is a sectional view of the thrombectomy apparatus along the A-A section according to the present embodiment; FIG. 12 is a sectional view of the balloon catheter according to the present embodiment. As shown in FIGs 11 and 12, the difference between this embodiment and the second embodiment is that the thrombectomy apparatus 1000 further includes an outer tube 1600 sleeved over the sheath 1500, a plurality of second perfusion channels 1610 are formed in the wall of the outer tube 1600 along the axial direction thereof, no first perfusion channel 1240 is formed in the balloon catheter 1200, and the second liquid is provided to the vicinity of the thrombus aspiration head 1300 only through the second perfusion channels 1610 by the second perfusion subunit 1820..

It should be understood that such arrangement can make the structure of the balloon catheter 1200 simpler, and can further reduce the manufacturing cost of the thrombectomy apparatus 1000.

In summary, embodiments of the present invention provide a thrombectomy apparatus, including a balloon catheter, a transmission tube, and a sheath. The balloon catheter is slidably inserted in the transmission tube, and the sheath is sleeved over the transmission tube, the distal and proximal ends of the balloon catheter are respectively connected with a balloon and a perfusion unit, and the distal end of the transmission tube and the distal end of the sheath are both connected with a thrombus aspiration head, the proximal end of the transmission tube and the proximal end of the sheath are respectively connected with a driving unit and an aspiration unit, and the space between the outer wall of the transmission tube and the inner wall of the sheath serves as a thrombus aspiration channel that is in communication with the thrombus aspiration head 1300. A filling channel is formed in the balloon catheter, the perfusion unit is configured to inject the first liquid into or draw the first liquid out of the balloon through the filling channel to expand or contract the balloon, and the perfusion unit is also configured to provide the second liquid to the vicinity of the thrombus aspiration head. The aspiration unit is configured to aspirate the thrombus into the thrombus aspiration head through the thrombus aspiration channel, and the driving unit is configured to drive the thrombus fragment cutter in the thrombus aspiration head to cut the thrombus into fragments that can be are aspirated out by the aspiration unit through the thrombus aspiration channel. In the thrombectomy apparatus provided by the present invention, the perfusion unit injects the first liquid into or draws the first liquid out of the balloon through the filling channel to expand or contract the balloon, so that the balloon and the inner wall of the blood vessel distal to the thrombus can be closely attached with each other. Therefore, the balloon can block the blood vessel distal to the thrombus during the thrombectomy, preventing thrombus fragments from escaping from the blood vessel distal to the thrombus. The perfusion unit further provides the second liquid to the vicinity of the thrombus aspiration head, and the second liquid may be a thrombolytic drug solution and/or normal saline, etc., which can be used to flush the thrombus to provide an additional impact thereon, thereby facilitating the detachment of the thrombus from the inner wall of the blood vessel. The thrombolytic drug solution can promote the dissolution of the thrombus. As a result, the operation time can be reduced, and the efficiency of the thrombectomy is improved. During the process of aspiration of the thrombus fragments, the thrombolytic drug solution and/or normal saline perfused in the vicinity of the thrombus aspiration head by the perfusion unit surrounds the thrombus fragments so as to be aspirated together by the aspiration unit, which can prevent the blood in the blood vessel from being aspirated by the aspiration unit excessively so as to reduce the blood loss of the patient.

The above are only preferred embodiments of the present invention, and do not restrict the present invention. Any person skilled in the art, without departing from the scope of the technical solution of the present invention, can make any form of equivalent replacement or modification or other changes to the technical solution and technical content disclosed by the present invention, which does not depart from the technical solution of the present invention and still falls within the protection scope of the present invention.

## Claims

1. A thrombectomy apparatus, comprising a balloon catheter, a transmission tube, and a sheath; wherein the balloon catheter is slidably inserted in the transmission tube, and the sheath is sleeved over the transmission tube;
wherein the balloon catheter has a distal end connected to a balloon, and a proximal end connected to a perfusion unit; the sheath has a distal end connected a thrombus aspiration head; the transmission tube is rotatably connected to the thrombus aspiration head; the transmission tube has a distal end fixedly connected with a thrombus fragmentation cutter accommodated in the thrombus aspiration head, and a proximal end connected to a driving unit; and the sheath further has a proximal end connected to an aspiration unit;
wherein a filling channel is formed in the balloon catheter; the perfusion unit is configured to inject a first liquid into or draw the first liquid out of the balloon through the filling channel to expand or contract the balloon; a first perfusion channel is formed in the balloon catheter; the first perfusion channel is provided with at least one first perfusion opening located between the balloon and the thrombus aspiration head; and the perfusion unit is further configured to provide a second liquid to an area between the balloon and the thrombus aspiration head through the first perfusion channel and the first perfusion opening;
wherein a space that serves as a thrombus aspiration channel is reserved between an outer wall of the transmission tube and an inner wall of the sheath; the thrombus aspiration channel is in communication with the thrombus aspiration head; the aspiration unit is configured to aspirate the thrombus into the thrombus aspiration head; the driving unit is configured to drive the thrombus fragmentation cutter to cut the thrombus into thrombus fragments; and the aspiration unit is configured to aspirate the thrombus fragments out through the thrombus aspiration channel.

2. The thrombectomy apparatus according to claim 1, wherein the balloon catheter is a multi-channel tube, and a guide wire channel is formed at a center of the balloon catheter.

3. The thrombectomy apparatus according to claim 2, wherein the filling channel and the first perfusion channel are symmetrical with each other about a central axis of the guide wire channel.

4. The thrombectomy apparatus according to claim 2, wherein an outer tube is sleeved over the sheath; at least one second perfusion channel is formed in the outer tube along an axial direction thereof; the second perfusion channel has at least one second perfusion opening located at a distal end of the outer tube; and the perfusion unit is further configured to provide a third liquid to an area between the balloon and the thrombus aspiration head through the second perfusion channel and the second perfusion opening.

5. The thrombectomy apparatus according to claim 4, wherein the distal end of the outer tube is horn-shaped.

6. The thrombectomy apparatus according to claim 1, wherein the perfusion unit comprises a first perfusion subunit and a second perfusion subunit; the first perfusion subunit is configured to inject the first liquid into the balloon or drawn out the first liquid from the balloon, and the second perfusion subunit is configured to provide the second liquid to the area.

7. The thrombectomy apparatus according to claim 6, wherein the first perfusion subunit comprises a first adaptor, a first infusion tube and a syringe that is provided therein with the first liquid; the first infusion tube has a distal end that is in communication with the filling channel via the first adaptor, and a proximal end that is in communication with the syringe.

8. The thrombectomy apparatus according to claim 6, wherein the second perfusion subunit comprises a second adaptor, a second infusion tube, and a perfusion liquid bag that is provided therein with the second liquid; the second infusion tube has a distal end that is in communication with the first perfusion channel via the second adaptor, and a proximal end that is in communication with the perfusion liquid bag; and a first peristaltic pump is provided to the second infusion tube.

9. The thrombectomy apparatus according to claim 1, wherein a plurality of thrombus aspiration holes are formed in a distal end and/or a sidewall of the thrombus aspiration head.

10. The thrombectomy apparatus according to claim 1, wherein the aspiration unit comprises a third adaptor, a third infusion tube, and a thrombus collection bag; the third infusion tube has a distal end that is in communication with the thrombus aspiration channel via the third adaptor, and a proximal end that is communication with the thrombus collection bag; a second peristaltic pump is provided to the third infusion tube.

11. The thrombectomy apparatus according to claim 1, wherein the first liquid is a developing solution, and the second liquid is a thrombolytic drug solution and/or normal saline.

12. A thrombectomy apparatus, comprising a balloon catheter, a transmission tube, a sheath, and an outer tube; wherein the balloon catheter is slidably inserted in the transmission tube, the sheath is sleeved over the transmission tube, and the outer tube is sleeved over the sheath;
wherein the balloon catheter has a distal end connected to a balloon, and a proximal end connected to a perfusion unit; the sheath has a distal end connected a thrombus aspiration head; the transmission tube is rotatably connected to the thrombus aspiration head; the transmission tube has a distal end fixedly connected with a thrombus fragmentation cutter accommodated in the thrombus aspiration head, and a proximal end connected to a driving unit; and the sheath further has a proximal end connected to an aspiration unit;
wherein a filling channel is formed in the balloon catheter; the perfusion unit is configured to inject a first liquid into or draw the first liquid out of the balloon through the filling channel to expand or contract the balloon; a plurality of second perfusion channels are formed in an wall of the outer tube along an axial direction thereof; a second perfusion opening is formed at a distal end of the outer tube; and the perfusion unit is further configured to provide a second liquid to an area between the balloon and the thrombus aspiration head through the second perfusion channel and the second perfusion opening;
wherein a space that serves as a thrombus aspiration channel is reserved between an outer wall of the transmission tube and an inner wall of the sheath; the thrombus aspiration channel is in communication with the thrombus aspiration head; the aspiration unit is configured to aspirate the thrombus into the thrombus aspiration head; the driving unit is configured to drive the thrombus fragmentation cutter to cut the thrombus into thrombus fragments; and the aspiration unit is configured to aspirate the thrombus fragments out through the thrombus aspiration channel.
